# EUROPÄISCHE PATENTSCHRIFT

(11) **EP 2 467 120 B1**
(45) Veröffentlichungstag und Bekanntmachung des Hinweises auf die Patenterteilung: **24.05.2017**
(21) Anmeldenummer: 10741970.7
(22) Anmeldetag: 12.08.2010
(51) Int. Cl.: A61K 8/02, A61K 8/34, A61K 8/97, A61Q 19/10, A61K 36/534, A61Q 19/00

(54) **ZUSAMMENSETZUNG ZUR REINIGUNG UND/ODER PFLEGE VON HAUT MIT EINER ETHANOLISCH-WÄSSRIGEN HYDRODISPERSIBLEN MINZKOMPOSITION**
COMPOSITION FOR CLEANING AND/OR CARING FOR THE SKIN, COMPRISING AN ETHANOLIC AND AQUEOUS HYDRODISPERSIBLE MINT COMPOSITION
COMPOSITION POUR LA PURIFICATION ET/OU LE SOIN DE LA PEAU CONTENANT UNE COMPOSITION DE MENTHE HYDRODISPERSIBLE, ÉTHANOLIQUE ET AQUEUSE

(30) Priorität: 20.08.2009 EP 09168291
(43) Veröffentlichungstag der Anmeldung: 27.06.2012
(73) Patentinhaber: Kneipp GmbH, 97084 Würzburg (DE)
(72) Erfinder: WOHLFART, Rainer, 97320 Sulzfeld am Main (DE); EBERT, Rebecca, 97852 Schöllbrunn (DE)
(74) Vertreter: Keller, Günter
(86) Internationale Anmeldenummer: PCT/EP2010/061721
(87) Internationale Veröffentlichungsnummer: WO 2011/020759

(56) Entgegenhaltungen:
- EP-A2- 1 281 396
- CN-A- 1 824 147
- DE-A1- 10 043 975
- DE-A1- 19 704 693
- JP-A- 9 077 636
- JP-A- 2000 229 836
- JP-A- 2003 192 568
- DATABASE WPI Week 200847 Thomson Scientific, London, GB; AN 2008-H42286 XP002570816, & KR 100 668 878 B1 (KANG M Y) 12. Januar 2007 (2007-01-12)
- DATABASE WPI Week 200821 Thomson Scientific, London, GB; AN 2008-C77447 XP002570820, & CN 1 994 270 A (UNIV SHANGHAI COMMUNICATION) 11. Juli 2007 (2007-07-11)
- "MENTHA PIPERITA (PEPPERMINT) EXTRACT & LEAF WATER", 1. Januar 2006 (2006-01-01), CTFA,, XP007911948, das ganze Dokument
- "KUHS KOSMETIK. DMS Concentrates", INTERNET CITATION, 2. Januar 2007 (2007-01-02), XP007911959, Gefunden im Internet: URL:http://web.archive.org/web/*/http://ku hs.com/data/dms_produkte.pdf [gefunden am 2010-02-26]
- DATABASE WPI Week 199845 Thomson Scientific, London, GB; AN 1998-529584 XP002570817, & RU 2 105 539 C1 (POPRAVKO S A) 27. Februar 1998 (1998-02-27)
- DATABASE WPI Week 200964 Thomson Scientific, London, GB; AN 2009-G09666 XP002570818, & KR 100 878 596 B1 (UNIV WOOSONG) 15. Januar 2009 (2009-01-15)
- DATABASE WPI Week 200923 Thomson Scientific, London, GB; AN 2009-F81895 XP002571044, & KR 2009 017 282 A (HANKOOK COSMETICS CO LTD) 18. Februar 2009 (2009-02-18)
- DATABASE WPI Week 200036 Thomson Scientific, London, GB; AN 2000-415183 XP002570819, & HU 9 800 538 A2 (SENDULA M) 28. Mai 2000 (2000-05-28)
- Kosmetik Konzept Koko Gmbh & Co: "Universal base creams with membrane structure for skin care, skin protection and dermatics", Österreichische Apothekerzeitung, Vol.56 (14), 1 January 2002 (2002-01-01), page 679, XP055104971, Retrieved from the Internet: URL:http://www.dermaviduals.com/cms/upload /Publikationen_english/OEAZ-14-2002-Basisc remes-engl.pdf [retrieved on 2014-02-28]

## Beschreibung

Gegenstand der vorliegenden Erfindung sind Zusammensetzungen, die zur Behandlung und/oder Pflege von Haut dienen. Durch die Fortschritte der Hygiene nehmen gerade in westlichen Industrieländern Allergien in weiten Teilen der Bevölkerung zu. Aufgrund des wachsenden Wohlstands und der Entwicklung bei der Körperpflege waschen und reinigen sich ein großer Teil der Bevölkerung häufig mehrmals am Tag gründlich und wenden dabei Hautreinigungs- und -pflegemittel, wie Waschlotionen, Hautöle und ähnliches an.

Bei den Produkten zur Reinigung und Pflege der Haut ist wesentlich, dass diese den hygienischen Vorschriften entsprechen und eine ausreichende Zeit der Lagerung ohne Beeinträchtigung (unerwünschte Geruchsveränderungen etc.) überstehen. Andererseits haben die zu diesem Zweck zugefügten Konservierungsmittel, ebenso wie Parfümöle und Duftstoffe die unerwünschte Eigenschaft, dass diese bei häufiger Anwendung der Hautreinigungs- und -pflegemittel von manchen Personen nicht gut vertragen werden, weil entweder schon Unverträglichkeiten hiergegen bestehen oder sich eine Sensibilisierung bis hin zu einer Kontaktallergie durch den häufigen Gebrauch entwickelt. Dies äußert sich meist durch Juckreiz, Rötungen, in schlimmeren Fällen Entzündungen und Hautausschläge, häufig verbunden mit starkem Juckreiz.

In dem Bereich der Körperpflege werden häufig Extrakte verschiedener Pflanzen, unter anderem auch Minze, eingesetzt. Die koreanische Patentanmeldung 100668878 beschreibt eine Zusammensetzung zur Förderung des Haarwachstums, wobei ethanolische Extrakte verschiedener Pflanzen, unter anderem auch Minze, eingesetzt werden. Die DE 100 43 975 schlägt die Verwendung eines Minzeextrakts bei einer wässrigen Lösung zur Fußpflege vor. Die RU 2105539 beschreibt eine Lotion für Hände und Gesicht mit natürlichem Honig, Alkohol und verschiedenen Kräuterextrakten, wobei auch Minze erwähnt ist. Die koreanische Patentanmeldung 100878596 beschreibt kosmetische Haarprodukte zur Verhinderung von Schuppen und Juckreiz, wobei ein Extrakt einer Mischung verschiedener Pflanzen, unter anderem auch Minze, eingesetzt wird. Die JP 2000229836 beschreibt ein kosmetisches Produkt zur Verhinderung der Hautalterung, das unter anderem auch einen Minzextrakt enthält. Die in der JP 09077636 beschriebene Präparation zur Aufhellung der Haut beinhaltet ethanolische Extrakte verschiedener Pflanzen, unter anderem auch von Pfefferminz.
Gegenstand der JP 2003/192568 ist eine wohlriechende Kosmetikzusammensetzung, wobei ein Parfüm durch Dampfdestillation einer wohlriechenden Pflanze hergestellt wird. Die CN 1994270 beschreibt ein Verfahren zur Dampfdestillation von wässrigen Extrakten aus verschiedenen duftintensiven Pflanzen wie Rosmarin, Thymus, Salbei, Eukalyptus und andere.
Die DE 197 04 693 A offenbart Hautpflegemittel in Form von Emulsionen oder Cremes, die natürliche Öle, Fette oder Wachse oder Ester linearer Fettsäuren und als Wirkstoffe Desoxyzucker oder diese enthaltende Naturstoffe enthalten.
Die EP 1 281 396 A betrifft eine hautvitalisierende Zusammensetzung zur äußeren Anwendung, um die Alterung der Haut zu vermindern, wobei verschiedenste Pflanzenextrakte eine Inhibitorwirkung auf Matrixmetallproteasen haben sollen.
Gemäß der KR 20090017282 sollen Extrakte verschiedener Pflanzen in kosmetischen Zusammensetzungen gegen die Hautalterung wirken, sowie Falten entgegenwirken und einen schützenden Effekt auf die Haut haben.
In der HU 9800538 wird offenbart, dass Wasser/Alkohol Extrakte verschiedener Pflanzen das Wachstum von Haar und die Regeneration von Haut fördern, sowie für die Verhinderung von Schuppen und Juckreiz eingesetzt werden können.
Auch in anderen Veröffentlichungen wird die Zugabe von Bestandteilen von Pfefferminzpflanzen zu kosmetischen Produkten vorgeschlagen. Bei diesen Veröffentlichungen wird häufig eine Mischung verschiedener Pflanzenbestandteile extrahiert und den kosmetischen Produkten zugegeben. Ein wesentlicher Nachteil dieser aus dem Stand der Technik bekannten Präparate ist, dass diese Pflanzenbestandteile beinhalten, die zu allergischen Reaktionen führen können.

Ausgehend davon besteht eine Aufgabe der Erfindung darin, Hautreinigungs- und -pflegemittel bereitzustellen, die einerseits frei sind von Substanzen mit sensibilisierender Wirkung, wie Konservierungsmitteln, Parfümölen, jedoch andererseits (auch ohne Konservierungsmittel) lagerstabil sind und (auch ohne Parfümöle und zum Zweck der Beduftung verwendeter Stoffe) die unangenehmen Eigengerüche von Hilfsstoffen, wie Tensiden oder Emulgatoren überdecken. Diese Hautreinigungs- und Hautpflegemittel sind für die topische Anwendung, also als Cremes, Öle, Lotionen u.ä. vorgesehen, nicht aber für die orale Verabreichung.

Gegenstand der vorliegenden Erfindung ist daher eine parfümölfreie Zusammensetzung zur Reinigung und/oder Pflege von Haut, die eine ethanolisch-wässrige hydrodispersible Minzkomposition in einer Menge von 0,005 - 0,5 Gew.-% bezogen auf das Gesamtgewicht der Zusammensetzung enthält, wobei die Minzkomposition derart hergestellt wurde, dass Pflanzen mit Wasserdampf extrahiert, die wässrige Phase aufkonzentriert und ethanolische Menthol-Lösung zugesetzt wurde, und die mehrere der folgenden Bestandteile: Triglyceride, Cholesterin, Phospholipide, freie Fettsäuren, Sqalen und/oder Ceramide enthält, die die Ausbildudung einer Derma-Membran-Struktur ermöglichen und bei der der Gehalt an Linalool der ethanolisch-wässrigen hydrodispersiblen Minzkomposition weniger als 1 Gew.-% bezogen auf die Minzkomposition beträgt. Aufgrund des erfindungsgemäßen Verfahrens wurde der Gehalt an potentiellen Allergenen auf ein Minimum reduziert.

Die erfindungsgemäße ethanolisch-wässrige hydrodispersible Minzkomposition wird durch eine Dampfdestillation von Teilen der frischen Pfefferminzpflanzen erhalten. Im Unterschied zu aus dem Stand der Technik bekannten Verfahren wird hier keine Mazeration oder Perkolation durchgeführt. Nach der Wasserdampfdestillation wird eine organische Phase erhalten, die einen Großteil des Menthols beinhaltet. Weiterhin wird eine wässrige Komponente erhalten, die erfindungsgemäß weiterverarbeitet wird. Durch eine entsprechende Aufarbeitung der wässrigen Fraktion werden potentielle allergene Stoffe abgereichert. Eine Abreicherung ist beispielsweise durch eine Ausschüttelung mit Octanol möglich. Die erfindungsgemäße ethanolisch-wässrige hydrodispersible Minzkomposition weist in bevorzugter Ausführungsform weniger als 0,5 Gew.-% und besonders bevorzugt weniger als 0,3 Gew.-% Linalool auf. Die Angaben in Gew.-% beziehen sich auf die Gesamtmenge an ethanolisch-wässriger hydrodispersibler Minzkomposition. Es wurde herausgefunden, dass der Gehalt an Linalool ohne die erfindungsgemäße Abreicherung bei bis zu etwa 6-7 %, bezogen auf die wässrige Phase, beträgt.

Einer der Vorteile der Verwendung der ethanolisch-wässrigen hydrodispersiblen Minzkomposition, im folgenden auch wässrige ewhM genannt, liegt darin, dass diese Komponente in einer verhältnismäßig geringen Konzentration zugegeben werden muß. Die ewhM wird bevorzugt in einer Menge zugegeben, die zwischen 0,005 und 0,1 Gew.-%, noch bevorzugter zwischen 0,01 und 0,08 Gew.-% liegt. Der Anteil bezieht sich auf das Verhältnis zwischen Gewicht der zugegebenen Fraktion und Gewicht des fertigen Produktes (Gewicht/Gewicht). Schon bei einem sehr geringen Anteil ewhM haben die Hautreinigungs- und -pflegemittel einen angenehmen Duft und eine kühlende, juckreizlindernde Wirkung.

Unter dem Begriff "hydrodispersibel" wird verstanden, dass die Zusammensetzung in wässriger Phase vollständig gelöst oder dispergiert werden kann. Um die Löslichkeit oder Dispergierbarkeit zu verbessern, kann ein Alkohol, wie Methanol, Propanol oder bevorzugt Ethanol zugesetzt werden.

In einer anderen bevorzugten Ausführungsform wird die ethanolisch-wässrige hydrodispersible Minzkomposition vor der Vermischung mit den anderen Komponenten der Zusammensetzung zuerst mit Ethanol vermischt. Die Menge des Ethanols in der fertigen Zusammensetzung kann 0-20 Gew.-%, bevorzugt 5-15 Gew.-% Ethanol betragen.

Die erfindungsgemäßen Zusammensetzungen zur Reinigung und/oder Pflege von Haut weisen in bevorzugter Ausführungsform keine Parfümöle auf. Unter Parfümölen werden Mischungen unverdünnter ätherischer Öle, natürlicher und/oder synthetischer einheitlicher Duftstoffe und/oder Mischungen dieser Komponenten verstanden, die zum Zweck der Beduftung zugesetzt und so zur Herstellung von Parfüm, Eau de Parfüm, Eau de Toilette oder zur Parfümierung von Kosmetika oder Haushaltsartikeln verwendet werden. Lediglich die ethanolisch-wässrige hydrodispersible Minzkomposition sorgt für einen dezenten, frischen Geruch wobei der bestimmungsgemäße Hauptzweck in der später noch eingehend beschriebenen kühlenden und juckreizlindernden Wirkung liegt. Außerdem weisen die erfindungsgemäßen Zusammensetzungen in bevorzugter Ausführungsform keine weiteren zum Zweck der Beduftung verwendeten Stoffe auf. Bei der ethanolisch-wässrigen hydrodispersiblen Minzkomposition handelt es sich nicht um ein Parfümöl, einen Duftstoff oder ein Konservierungsmittel im Sinne dieser Definition.

Die erfindungsgemäßen Zusammensetzungen können eine Derma-Membran-Struktur ausbilden. Bei der Derma-Membran-Struktur werden vor allem Triglyceride, Cholesterin, Phospholipide, freie Fettsäuren, Squalen und Ceramide eingesetzt. Hierbei handelt es sich um hautverwandte Lipide pflanzlichen Ursprungs, die zur Lipidsubstitution gerade bei geschädigter Hautbarriere besonders geeignet sind. In bevorzugter Ausführungsform weisen die erfindungsgemäßen Zusammensetzungen keine Paraffine und/oder Silikone auf, da diese okklusiv wirkenden Verbindungen den pflegenden Charakter der erfindungsgemäßen Zusammensetzungen eher stören.

Die erfindungsgemäßen Zusammensetzungen sind vor allem für die Anwendung bei solchen Personen geeignet, die Neigungen zu Hautallergien haben oder die unter Erkrankungen der Haut leiden. Derartige Erkrankungen können beispielsweise die Schuppenflechte sein, aber auch trockene Haut und Schädigungen der Haut, die beispielsweise durch berufliche Tätigkeiten, nämlich den häufigen Kontakt mit reizenden Substanzen hervorgerufen werden.

Besonders vorteilhaft bei den erfindungsgemäßen Zusammensetzungen sind die Verringerung des Juckreizes (Antipruritus-Wirkung) und die kühlende Wirkung.

Die ethanolisch-wässrige hydrodispersible Minzkomposition enthält zwar einen Anteil Menthol, das in einer Konzentration ab 0,1 % eine Antipruritus-Wirkung hat (Patri, F.; Silano V.; Plants in Cosmetics, Council of Europe Publishing, Strasbourg (2002). Steinegger, E., Hänsel. R.; Lehrbuch der Pharmakognosie und Phythopharmazie, Springer Verlag Berlin (1988). Die Juckreiz vermindernde Wirkung der ethanolisch-wässrigen hydrodispersiblen Minzkomposition übertrifft die Wirkung des Menthols um ein Vielfaches und setzt bereits ab einer Menge von 0,005 Gew.-% der ewhM (Gewicht/Gewicht) ein. Durch Zusatz einer geringen Ethanolmenge (1-20 %, bevorzugt 2-15 %, besonders bevorzugt 4-12 % Ethanol, bezogen auf die fertige Zusammensetzung) kann dieser Effekt noch intensiviert werden. Für die Antipruritus-Wirkung ist daher nicht oder nicht nur der Mentholbestandteil ausschlaggebend, sondern die anderen Verbindungen, die in der wässrigen Minzölfraktion enthalten sind.

Schon durch die geringen Konzentrationen der ethanolisch-wässrigen hydrodispersiblen Minzkomposition kann der Zusammensetzung ein angenehmer und dauerhaft stabiler Minzduft verliehen werden. Die erfindungsgemäß eingesetzten Konzentrationen der ethanolisch-wässrigen hydrodispersiblen Minzkomposition liegt im Vergleich zu konventionellen ätherischen Ölen und/oder anderen Parfümölen, die üblicherweise Hautpflege- bzw. Hautbehandlungsprodukten zugefügt werden, um etwa eine Zehnerpotenz niedriger.

Aufgrund der geringen Konzentrationen an ewhM treten selbst bei Personen mit empfindlicher Haut keine Unverträglichkeitsreaktionen auf, wie in einer Anwendungsstudie mit Heimapplikation an 75 Probanden/-innen der Universität Osnabrück, Fachbereich Humanwissenschaften, Dermatologie, Umweltmedizin und Gesundheitstheorie bestätigt.

Die Studie wurde an Probanden, 55 weiblich und 20 männlich, 31 ±13 Jahre alt, durchgeführt, die folgende Charakteristika aufwiesen:
- "Empfindliche Haut", d.h. eine anlagebedingte oder erworbene hohe Reaktionsbereitschaft auf Externa (Creme/Lotion/Salbe, Make-up, Wasch-/Pflege-/Reinigungsmittel, etc.) mit Symptomen, wie z.B. Jucken, Brennen, Rötung oder Schuppung.
- "Atopische Diathese", d.h. Neigung zu Heuschnupfen und / oder Neurodermitis

Dabei wurde folgendes Ergebnis erreicht:
- Die Verträglichkeit der Testprodukte ist als "sehr gut" zu beurteilen.
- Das Einziehverhalten der Testprodukte (Pflegeprodukte) ist als "gut" zu beurteilen.
- Das Hautgefühl nach der Anwendung der Testprodukte ist als "gut" zu beurteilen.
- Der Geruch ist als "gut" - "befriedigend" zu beurteilen.

Ein weiterer Vorteil der Zugabe der ethanolisch-wässrigen hydrodispersiblen Minzkomposition ist, dass keine allergenen Stoffe, die im Sinne der Kosmetik-Verordnung deklarationspflichtig sind, zugegeben werden müssen.

Die erfindungsgemäß eingesetzte, ethanolisch-wässrige hydrodispersible Minzkomposition wird vorzugsweise derart hergestellt, dass
1. frisches oder getrocknetes, teilweise zerkleinertes Pflanzenmaterial, nämlich vor allem Blätter, aber auch Stengel von verschiedenen Sorten der Pfefferminzpflanze in ein geeignetes Gefäß verbracht werden und von unten bedampft werden. Der Wasserdampf löst aus den Pflanzenteilen die flüchtigen Bestandteile, vor allem essenzielle Öle heraus. Dieser mit den gewünschten Pflanzenbestandteilen gesättigte Wasserdampf wird über eine geeignete Kühlvorrichtung abgekühlt und in einem geeigneten Gefäß (Tank) abkühlen gelassen. Dabei scheidet sich oben die organische Phase mit den Ölbestandteilen und unten die wässrige Phase ab.
2. die wässrige Phase wird aufkonzentriert. Bei diesem Verfahrensschritt kann auch in bevorzugter Ausführungsform eine Fraktionierung stattfinden, insbesondere durch Ausschütteln mit verschiedenen organischen Lösungsmitteln, wie Alkoholen, insbesondere Octanol, Ketonen oder Kohlenwasserstoffen.
3. eine ethanolische Menthol-Lösung wird zugesetzt. So entsteht die ethanolisch-wässrige hydrodispersible Minzkomposition. Diese zeichnet sich überraschenderweise dadurch aus, dass sie bereits in geringer Menge (0,01 - 0,07%) eine juckreizmildernde und stark kühlende Wirkung entfaltet. Der Vorteil dieser geringen Wirkstoffkonzentration liegt darin dass im Unterschied zu Menthol auch bei hautsensiblen Personen nachweislich keine unangenehmen Hautreaktionen auftreten. Ein erwünschter Nebeneffekt der Komposition ist, der angenehme Geruch, der unangenehme Eigengerüche der Hilfstoffe überdeckt ohne selbst die Definition eines Parfümöls zu erfüllen.

**Tabelle 1: Zusammensetzung der ethanolisch wässrigen hydrodispersiblen Minzkomposition (ewhM). Die mittlere Spalte der Tabelle 1 gibt die jeweiligen Bestandteile der ewhM an, die rechte Spalte den Anteil der einzelnen Komponenten in dem fertigen Produkt.**

| **Chemische Verbindung** | **Anteil der ewhM in Gew**.**-%** | **Anteil in Gew.-% in bevorzugten Ausführungsformen** |
|---|---|---|
| Ethanol/Ethylalcohol | 10 - 20 | 0,002 - 0,007 |
| Menthol | 20 - 30 | 0,004 - 0,021 |
| wässrige Minzöl-Fraktion | 40 - 50 | 0,008 - 0,035 |

Die gaschromatographische Analyse der wässrigen Minzöl-Fraktion, Tabelle 2, der ewhM, Tabelle 3, und die vergleichende Untersuchung einer Probe ätherischen Pfefferminzöls, Tabelle 4, zeigt quantitative Unterschiede in den Zusammensetzungen.

Außerdem fehlen in der wässrigen Minzöl-Fraktion und im ewhM die Komponenten alpha-Pinen und beta-Pinen, die für ein Pfefferminzöl typisch sind. Ein sekundäres Oxidationsprodukt von Pinen ist das allergieauslösende Ascanidol. Da die erfindungsgemäße Minzkomposition weniger als 0,5, bevorzugt weniger als 0,3 und besonders bevorzugt weniger als 0,1 Gew.-% α- und/oder β-Pinen, bezogen auf die eingesetzte Minzkomposition, aufweist, beinhaltet dieses Präparat keine von Ascanidol abgeleiteten allergenen Verbindungen.

**Tabelle 2: Zusammensetzung einer erfindungsgemäßen wässrigen Minzöl-Fraktion (GC-Analyse) vor Zugabe des Mentholanteils**

| Ingredient | Cas | Amount |
|---|---|---|
| Menthone | 14073-97-3 | 26,04% |
| Menthofurane | 494-90-6 | 9,99% |
| Menthyl Acetate | 16409-45-3 | 8,00% |
| Isomenthone | 491-07-6 | 4,50% |
| Pulegone | 3 285-04-9 | 4,12% |
| 1,8 Cineole | 470-85-6 | 3,10% |
| Beta-Caryophyllene | 87-44-5 | 3,03% |
| Terpinen-4-Ol | 562-74-3 | 2,78% |
| Neo-Isomenthol | 491-02-1 | 1,83% |
| Germacrene-D | 37839-63-7 | 1,06% |
| Piperitone | 6091-50-5 | 0,80% |
| Isomenthyl Acetate | 20777-45-1 | 0,48% |
| Beta-Bourbonene | 5208-59-3 | 0,37% |
| Limonene | 5989-27-5 | 0,23% |
| Linalool | 78-70-6 | 0,22% |

**Tabelle 3: Zusammensetzung einer erfindungsgemäßen ethanolisch-wässrigen hydrodispersiblen Minzölkomposition (GC-Analyse) nach Zugabe von Menthol**

| Ingredient | Cas | Amount |
|---|---|---|
| Menthol | 2216-51-5 | 65,13% |
| Menthone | 14073-97-3 | 9,08% |
| Menthofurane | 494-90-6 | 3,45% |
| Menthyl Acetate | 16409-45-3 | 2,79% |
| Isomenthone | 491-07-6 | 1,57% |
| Pulegone | 3 285-04-9 | 1,46% |
| 1,8 Cineole | 470-85-6 | 1,08% |
| Beta-Caryophyllene | 87-44-5 | 1,06% |
| Terpinen-4-Ol | 562-74-3 | 0,97% |
| Neo-Isomenthol | 491-02-1 | 0,64% |
| Germacrene-D | 37839-63-7 | 0,37% |
| Piperitone | 6091-50-5 | 0,28% |
| Isomenthyl Acetate | 20777-45-1 | 0,17% |
| Beta-Bourbonene | 5208-59-3 | 0,13% |
| Limonene | 5989-27-5 | 0,08% |
| Linalool | 78-70-6 | 0,07% |

**Tabelle 4: Zusammensetzung einer Vergleichsprobe ätherischen Pfefferminzöls (GC-Analyse)**

| Ingredient | Cas | Amount |
|---|---|---|
| Menthol | 2216-51-5 | 47,00% |
| Menthone | 14073-97-3 | 20,50% |
| Menthyl Acetate | 16409-45-3 | 5,00% |
| 1,8 Cineole | 470-85-6 | 4,40% |
| Menthofurane | 494-90-6 | 3,00% |
| Isomenthone | 491-07-6 | 2,90% |
| Pulegone | 3 285-04-9 | 2,20% |
| Beta-Caryophyllene | 87-44-5 | 1,60% |
| Limonene | 5989-27-5 | 1,50% |
| β-Pinen | 127-91-3 | 0,70% |
| Germacrene-D | 37839-63-7 | 0,70% |
| α-Pinen | 7785-70-8 | 0,50% |
| Piperitone | 6091-50-5 | 0,50% |
| P-Cymene | 99-87-6 | 0,40% |
| Sabinene Hydrate | 546-79-2 | 0,40% |
| Sabinene | 3387-41-5 | 0,40% |

Je nach Anwendungsgebiet können die erfindungsgemäßen Zusammensetzungen nach den folgenden bevorzugten Vorschriften hergestellt werden:

### Herstellungsvorschriften

### I) Creme

Eine erfindungsgemäße Creme, die insbesondere für die Anwendung am Gesicht geeignet ist, kann beispielsweise so hergestellt werden, dass zunächst eine Wasserphase hergestellt wird, die neben Wasser, feuchtigkeitsspendende Stoffe, wie Dextrin, Glycerin, sowie gegebenenfalls Mittel zur Einstellung des pH-Wertes wie Citronensäure enthält.

Weiterhin wird die Fettphase, die Öle, Wachse, Sheabutter und weitere Komponenten wie mikrokristalline Zellulose enthält, auf 70-80°C erwärmt und gut gerührt. Dann wird die Wasserphase langsam unter die Fettphase gezogen, wobei eine Mischung der Fett- und der Wasserphase durch Verwendung eines Homogenisators bewirkt werden kann. Die homogenisierte Masse kann dann langsam abgekühlt werden und es wird der pH-Wert auf den gewünschten Bereich eingestellt. Üblicherweise liegt der pH-Wert bei Cremes in dem Bereich zwischen 4,0 und 6,5, bevorzugt 4,5 bis 5,2, besonders bevorzugt bei pH 5,0. Damit das fertige Produkt eine Derma-Membranstruktur aufweist, wird DMS Probiol N 03015 (Kuhs GmbH & Co. Laboratorien, Langfeld, Deutschland) bei einer Temperatur zwischen etwa 25°C und 27°C unter Homogenisierungsbedingungen eingearbeitet.

Anschließend wird die ewhM (Mint Natural Hydrodispersible, Robertet GmbH, Köln, Deutschland), gegebenenfalls vermischt mit Ethanol, zugegeben und anschließend wird die ganze Mischung nochmals homogenisiert. Dann kann die Mischung abgekühlt werden. Es wird eine homogene, gut fließfähige Emulsion erhalten, deren Oberfläche glänzend ist.

### II) Körperlotion

Eine erfindungsgemäße Körperlotion kann beispielsweise so hergestellt werden, dass zunächst eine Wasserphase hergestellt wird, die der Fettphase zugefügt wird.

Dazu wird zuerst Wasser sterilisiert, anschließend auf 60°C abgekühlt. Glycerol 85 %, Dextrin gelöst in Wasser bei 60°C, wasserfreie Citronensäure, ebenfalls gelöst in Wasser bei 60°C, werden unter Rühren bei laufendem Homogenisator (2000U/min) über den Homogenisator eingearbeitet. Bei 70°C wird die Fettphase bestehend aus: Jojobawachs, Sheabutter, Camelinaöl 3/6, Verdickern und Vitaminen, unter Rühren in die Fryma gezogen (Homogenisator bei 2000U/min) und dann homogenisiert bei 3000U/min, 4min über die Ringleitung. Unter Rühren wird auf max. 30°C abgekühlt. Dann wird eine Vormischung aus Ethanol- / Wasser- / ewhM (Mint Natural Hydrodispersible, Robertet GmbH, Köln, Deutschland) und DMS Probiol N 03015 (Kuhs GmbH & Co. Laboratorien, Langfeld, Deutschland) bei Raumtemperatur unter Rühren eingezogen. Anschließend wird über die Ringleitung homogenisiert (2100 U/min).

### III) Hautöl

Zunächst wird ein geeignetes Pflanzenöl, wie beispielsweise Sonnenblumenöl vorgelegt und mit Jojobawachs, Camelinaöl 3/6, Vitaminen und der wässrigen ewhM (Mint Natural Hydrodispersible, Robertet GmbH, Köln, Deutschland) vermischt. Diese Mischung wird gerührt, bis sie klar wird. Dies tritt nach ca. 10-15 Min. ein.

### IV) Waschlotion

Zunächst wird Wasser und Glycerin in einem Mischgefäß vorgelegt. Dann wird Natrium-Cocoyl-Glutamat (Plantacare ACG HC, Cognis GmbH, Monheim am Rhein, Deutschland) zugefügt. Anschließend wird mit der Öl-/Verdickerphase auf Basis von Jojobawachs vermischt. Die Phasen werden langsam bis zu Homogenität vermischt. Dabei wird die Phase milchig. Anschließend wird die Ethanolphase, bestehend aus Ethanol, wässriger ewhM (Mint Natural Hydrodispersible, Robertet GmbH, Köln, Deutschland) und Glyceryl Caprylate (Dermosoft GMCY, Dr. Straetmans GmbH, Hamburg, Deutschland) eingerührt, bis eine homogene Mischung entsteht. Während der Neutralisationsphase wird Wasser und Citronensäure zuerst gemischt und dann zugeführt. Nach Mischung für 15 -20 Minuten mit dem Homogenisator kann die fertige Lotion entnommen werden.

Die erfindungsgemäßen Zusammensetzungen zur Pflege und/oder Reinigung der menschlichen Haut haben verschiedene überraschende Vorteile. Einerseits können kosmetische Präparate bereitgestellt werden, die frei sind von Parfümölen, und/oder Konservierungsstoffen und lediglich die ethanolisch-wässrige hydrodispersible Minzkomposition in einer sehr geringen Menge aufweisen. Diese Menge liegt bevorzugt zwischen 0,005 und 0,5 Gew.-%, bezogen auf das Gesamtgewicht der Zusammensetzung. Daher sind die erfindungsgemäßen Produkte besonders für Personen mit Hautallergie, trockener Haut und besonders empfindlicher Haut geeignet.

Ein weiterer Vorteil der Zugabe der ethanolisch-wässrigen hydrodispersiblen Minzkomposition liegt darin, dass diese Komponente eine Juckreiz stillende Wirkung aufweist. Das bedeutet, dass durch die Anwendung der erfindungsgemäßen Produkte der Juckreiz gelindert wird. Dies ist vor allem bei Allergikern oder auch bei Personen mit trockener Haut oder mit einschlägigen Erkrankungen, wie Schuppenflechte besonders vorteilhaft. Schließlich vermittelt die erfindungsgemäß eingesetzte ethanolisch-wässrige hydrodispersible Minzkomposition einen dezenten Wohlgeruch, der den unaufdringlichen Eindruck von Frische vermittelt.

Die einzelnen Komponenten der fertigen Produkte variieren hinsichtlich ihrer jeweiligen Bestandteile innerhalb gewisser Grenzen. In der nachfolgenden Tabelle 1 sind die wesentlichen Komponenten der jeweiligen Produkte mit bevorzugten Bereichen angegeben.

**Tabelle 5: Bevorzugte Bereiche der einzelnen Komponenten in den fertigen Produkten**

| **Herstellungsbeispiel** | **Creme 1** | **Körperlotion 2** | **Hautöl 3** | **Waschlotion 4** |
|---|---|---|---|---|
| **Wasser** | **35-55** | **55-70** | **0-10** | **45-70** |
| **Pflanzenöl** | **0-10** | **0-10** | **45-65** | **0** |
| **DMS** | **20-50** | **5-20** | **0** | **0-5** |
| **Sheabutter** | **7,5-10** | **2-3** | **0-5** | **0-5** |
| **Ethanol 96 %** | **5-10** | **5-10** | **0-5** | **5-15** |
| **Camelina Öl 3/6** | **2-10** | **2-10** | **5-25** | **1-20** |
| **Jojoba-Wachs** | **2-10** | **2-10** | **25-35** | **4-6** |
| **Sodium Cocoyl Glutamate** | **0** | **0** | **0** | **10-50** |
| **Glycerol 85 %** | **3-10** | **3-10** | **0** | **3-10** |
| **Wässrige Minzöl-Fraktion** | **0,005-0,5** | **0,005-0,5** | **0,005-0,5** | **0,005-0,5** |

Die Mengenangaben in Gewichtsprozent sind bezogen auf das fertige Produkt. Die Angaben ergänzen sich durch übliche Inhaltsstoffe von Kosmetika, wie Mitteln zur Einstellung der Viskosität, wie Xanthan, und Carrageenan, pH-Wert-Regulierern, wie Citronensäure, Vitamine, z.B. Vitamin E in Form von Mischtocopherol 70% (natürlich), etc. zu 100 Gew%.

Die nachfolgenden Beispiele erläutern die vorliegende Erfindung, wobei dem Fachmann bekannt ist, dass derartige Zusammensetzungen ohne Schwierigkeiten hergestellt werden können.

### Beispiel 1

Es wurde eine Creme gemäß dem Herstellungsbeispiel 1 hergestellt.

Der Rezeptur kommt nachweislich eine barriereregenerierende Wirkung zu. Es wurde eine Studie an 21 Personen durchgeführt.

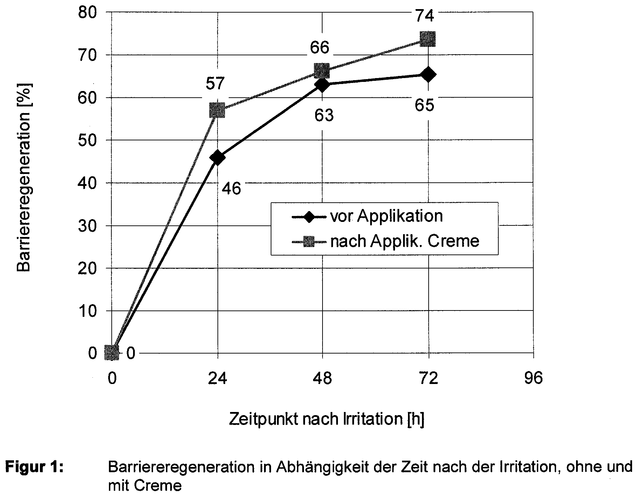

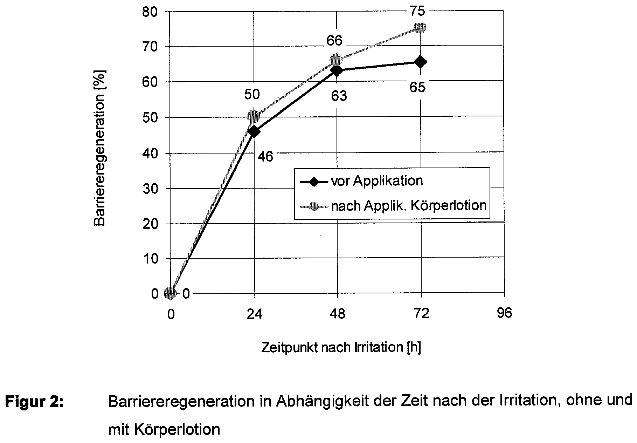

Die Barriereregeneration einer mechanisch geschädigten Haut wird durch die Applikation der Creme gemäß Herstellungsbeispiel 1 unterstützt. Dieser unterstützende Effekt ist bereits 24h nach der Barriereschädigung messbar und verbleibt über die Dauer von 72h nach der Barriereschädigung. Die Ergebnisse sind in Figur 1 dargestellt.

### Beispiel 2

Es wurde eine Körperlotion gemäß Herstellungsbeispiel 2 hergestellt.

Die Barriereregeneration einer zuvor geschädigten Haut wird durch die Applikation der Körperlotion gemäß Beispiel 2 unterstützt. Dieser unterstützende Effekt ist erst 72h nach der Barriereschädigung, dann aber sehr deutlich messbar.

In der Studie wurde für die beiden Testprodukte ein unterstützender Effekt auf die Barriereregeneration nach Schädigung durch Tesafilmabrisse im Vergleich zu einer unbehandelten Kontrolle nachgewiesen.

Für beide Testprodukte gilt: Sie unterstützen die Barriereregeneration der Haut nach mechanischer Schädigung.

## Patentansprüche

1. Parfümölfreie Zusammensetzung zur Reinigung und/oder Pflege von Haut, die eine ethanolisch-wässrige hydrodispersible Minzkomposition in einer Menge von 0,005 - 0,5 Gew.-% bezogen auf das Gesamtgewicht der Zusammensetzung enthält, wobei die Minzkomposition derart hergestellt wurde, dass Pflanzen mit Wasserdampf extrahiert, die wässrige Phase aufkonzentriert und ethanolische Menthol-Lösung zugesetzt wurde, **dadurch gekennzeichnet, dass** diese mehrere der folgenden Bestandteile: Triglyceride, Cholesterin, Phospholipide, freie Fettsäuren, Sqalen und/oder Ceramide enthält, die die Ausbildung einer Derma-Membran-Struktur ermöglichen und, dass der Gehalt an Linalool der ethanolisch-wässrigen hydrodispersiblen Minzkomposition weniger als 1 Gew.-% bezogen auf die Minzkomposition beträgt.

2. Zusammensetzung nach Anspruch 1 , **dadurch gekennzeichnet, dass** der Gehalt an α- und/oder β-Pinen weniger als 1,0 Gew.-% bezogen auf die eingesetzte MinzölKomposition beträgt.

3. Zusammensetzung nach einem der Ansprüche 1 oder 2, **dadurch gekennzeichnet, dass** die Zusammensetzung 0,01 - 0,08 Gew.-% einer ethanolisch-wässrigen hydrodispersiblen Minzkomposition bezogen auf das Gesamtgewicht der Zusammensetzung enthält.

4. Zusammensetzung nach einem der vorhergehenden Ansprüche, **dadurch gekennzeichnet, dass** diese keine weiteren zum Zweck der Beduftung verwendeten Stoffe auf und/oder Konservierungsmittel enthält.

5. Zusammensetzung nach einem der vorhergehenden Ansprüche, **dadurch gekennzeichnet, dass** diese maximal 20 Gew.-% Ethanol enthält.

6. Zusammensetzung nach einem der Ansprüche 1 bis 5, **dadurch gekennzeichnet, dass** es eine Creme ist.

7. Zusammensetzung nach einem der Ansprüche 1 bis 5, **dadurch gekennzeichnet, dass** es eine Körperlotion ist.

8. Zusammensetzung nach einem der Ansprüche 1 bis 4, **dadurch gekennzeichnet, dass** es eine Waschlotion ist.

9. Zusammensetzung nach einem der vorhergehenden Ansprüche, **dadurch gekennzeichnet, dass** diese für die Anwendung bei Personen mit Neigung zu hochsensibler/trockener Haut oder Hautallergien geeignet ist.

10. Verwendung einer Zusammensetzung gemäß einem der Ansprüche 1 bis 8 zur Reinigung und/oder Pflege der Haut.

11. Zusammensetzung nach einem der Ansprüche 1 bis 8 zur Anwendung bei der Reinigung der Haut von Menschen, die an trockener Haut und Hautallergien leiden.

12. Zusammensetzung nach einem der Ansprüche 1 bis 8 zur Anwendung bei der Pflege der Haut von Menschen, die an trockener Haut und Hautallergien leiden.

13. Zusammensetzung zur Anwendung nach einem der Ansprüche 11 oder 12 wobei es sich bei der Erkrankung der Haut um die Schuppenflechte handelt.

## Claims

1. A perfume oil-free composition for cleansing and/or care of skin, containing an ethanolic-aqueous hydrodispersible mint composition in an amount of 0.005 - 0.5 wt %, based on the total weight of the composition, wherein the mint composition is prepared by extracting plants with water vapour, concentrating the aqueous phase, and adding ethanolic menthol solution, **characterized in that** said composition contains a plurality of the following ingredients: triglycerides, cholesterol, phospholipides, free fatty acids, squalene and/or ceramides allowing formation of a derma membrane structure and **in that** the linalool content of the ethanolic-aqueous hydrodispersible mint composition is less than 1 wt %, based on the mint composition.

2. The composition according to claim 1, **characterized in that** the content of α- and/or β-pinene is less than 1.0 wt %, based on the mint oil composition used.

3. The composition according to claims 1 or 2, **characterized in that** said composition contains 0.01 - 0.08 wt % of an ethanolic-aqueous hydrodispersible mint composition, based on the total weight of the composition.

4. The composition according to any of the preceding claims, **characterized in that** said composition does not contain any further substances used for scenting and/or does not contain any preservative agents.

5. The composition according to any of the preceding claims, **characterized in that** said composition contains a maximum of 20 wt % of ethanol.

6. The composition according to any of claims 1 to 5, **characterized in that** said composition is a cream.

7. The composition according to any of claims 1 to 5, **characterized in that** said composition is a body lotion.

8. The composition according to any of claims 1 to 4, **characterized in that** said composition is a washing lotion.

9. The composition according to any of the preceding claims, **characterized in that** said composition is suitable for the application with persons being susceptible to highly sensitive/dry skin or cutaneous allergies.

10. Use of a composition according to any of claims 1 to 8 for cleansing and/or care of skin.

11. The composition according to any of claims 1 to 8 for use in cleansing skin of humans, suffering from dry skin and cutaneous allergies.

12. The composition according to any of claims 1 to 8 for use in the care of skin of humans, suffering from dry skin and cutaneous allergies.

13. A composition for use according to any of claims 11 or 12, wherein the skin disease is psoriasis.

## Revendications

1. Composition sans huile de parfum pour la purification et/ou le soin de la peau, qui contient une composition de menthe hydrodispersible, éthanolique et aqueuse en une quantité de 0,005 - 0,5 % en poids par rapport au poids total de la composition, dans laquelle la composition de menthe a été préparée de telle manière que des plantes ont été extraites à la vapeur d'eau, la phase aqueuse a été concentrée et une solution de menthol éthanolique a été ajoutée, **caractérisée en ce que** celle-ci contient plusieurs des composants suivantes : triglycérides, cholestérol, phospholipides, acides gras libres, squalène et/ou céramide, qui permettent la formation d'une structure derma membrane et, **en ce que** la teneur en linalol de la composition de menthe hydrodispersible, éthanolique et aqueuse est inférieure à 1 % en poids par rapport à la composition de menthe.

2. Composition selon la revendication 1, **caractérisée en ce que** la teneur en α-pinène et/ou ß-pinène est inférieure à 1,0 % en poids par rapport à la composition d'huile de menthe utilisée.

3. Composition selon l'une quelconque des revendications 1 ou 2, **caractérisée en ce que** la composition contient 0,01 - 0,08 % en poids d'une composition de menthe hydrodispersible, éthanolique et aqueuse par rapport au poids total de la composition.

4. Composition selon l'une quelconque des revendications précédentes, **caractérisée en ce que** celle-ci ne contient aucune autre substance utilisée à des fins de parfumage et/ou conservateur.

5. Composition selon l'une quelconque des revendications précédentes, **caractérisée en ce que** celle-ci contient au maximum 20 % en poids d'éthanol.

6. Composition selon l'une quelconque des revendications 1 à 5, **caractérisée en ce qu'**il s'agit d'une crème.

7. Composition selon l'une quelconque des revendications 1 à 5, **caractérisée en ce qu'**il s'agit d'une lotion corporelle.

8. Composition selon l'une quelconque des revendications 1 à 4, **caractérisée en ce qu'**il s'agit d'une lotion lavante.

9. Composition selon l'une quelconque des revendications précédentes, **caractérisée en ce que** celle-ci convient pour l'application chez les personnes qui ont tendance à avoir une peau très sensible/sèche ou des allergies cutanées.

10. Utilisation d'une composition selon l'une quelconque des revendications 1 à 8 pour la purification et/ou le soin de la peau.

11. Composition selon l'une quelconque des revendications 1 à 8 pour l'application lors de la purification de la peau de personnes qui présentent une peau sèche et des allergies cutanées.

12. Composition selon l'une quelconque des revendications 1 à 8 pour l'application lors du soin de la peau de personnes qui présentent une peau sèche et des allergies cutanées.

13. Composition pour l'application selon l'une quelconque des revendications 11 ou 12, dans laquelle la maladie de la peau est le psoriasis.
